# EUROPEAN PATENT APPLICATION

(11) **EP 4 548 851 A1**
(43) Date of publication of application: **07.05.2025**
(21) Application number: 23207344.5
(22) Date of filing: 02.11.2023
(51) Int. Cl.: A61B 6/03, A61B 6/00, A61B 6/42

(54) **SPECTRAL RESCUE FOR CONTRASTED CT EXAMS**

(71) Applicant: Koninklijke Philips N.V., 5656 AG Eindhoven (NL)
(72) Inventor: HARDER, Tim Philipp, Eindhoven (NL); BUELOW, Thomas, Eindhoven (NL); DESHPANDE, Hrishikesh Narayanrao, 5656AG Eindhoven (NL); NORDHOFF, Tanja, Eindhoven (NL); SCHMITT, Holger, Eindhoven (NL)
(74) Representative: Philips Intellectual Property & Standards

(57) **Abstract**

In order to improve contrast-enhanced CT exams, an image quality assurance device (10) is provided. The image quality assurance device (10) comprises an input unit (12), a processing unit (14), and an output unit (16). The input unit is configured to receive image data acquired by a spectral Computed Tomography (CT) scanner. The processing unit is configured to perform an analysis of a contrast enhancement in a region of interest in the acquired image data, and to compare the contrast enhancement of the region of interest to a desired contrast enhancement as defined by an exam protocol to determine whether the contrast enhancement of the region of interest is sufficient. In response to determining that there is an insufficient contrast enhancement of the region of interest, the processing unit is configured to generate a trigger signal, which is usable to trigger the spectral CT scanner to export spectral data. The output unit is configured to output the generated trigger signal.

## Description

### FIELD OF THE INVENTION

The present invention relates to contrast Computed Tomography (CT) exams. In particular, the present invention relates to an image quality assurance device, to an imaging system, to an image quality assurance method, to a computer program product, and to a computer-readable medium.

### BACKGROUND OF THE INVENTION

Contrast-enhanced CT exams account for about 50% of all CT acquisitions. In the contrast-enhanced CT exams, contrast materials, also known as contrast agents or contrast media, are used to improve the diagnostic value of those imaging exams. Iodine-based and barium-sulfate compounds are typically used in x-ray and CT imaging exams. When iodine-based and barium-sulfate contrast materials are present in a specific area of the body, they block or limit the ability of x-rays to pass through. As a result, blood vessels, organs and other body tissue that temporarily contain the iodine-based or barium compounds change their appearance on x-ray or CT images. The contrast-enhanced CT exams are more involved from the clinical workflow due to the additional steps required and complex scan parameters to be considered. Depending on the clinical question and target anatomy, the iodine needs to be in specific parts of the patient's tissue or vessels to lead to the desired enhancement and, subsequently, allow for a confident diagnosis. Modern spectral CT systems record the radiation at two energy levels and have the ability to retrospectively split the detected radiation in various energy bins. This allows to produce so called mono-energetic reconstructions at lower energy levels resulting in a stronger iodine contrast enhancement effect. In clinical routine, this allows to use lower quantities of contrast agent when performing spectral acquisitions.

Unfortunately, even though the scanner has spectral capabilities, many of the scans performed on those machines are not set up to record the spectral information along with the imaging data, basically throwing away the ability to reconstruct mono-energetic images.

### SUMMARY OF THE INVENTION

There may be a need to improve contrast-enhanced CT exams.

The object of the present invention is solved by the subject-matter of the independent claims, wherein further embodiments are incorporated in the dependent claims. It should be noted that the following described aspects of the invention apply also for the image quality assurance device, the imaging system, the image quality assurance method, the computer program product, and the computer-readable medium.

According to a first aspect of the present invention, there is provided an image quality assurance device. The image quality assurance device comprises an input unit, a processing unit, and an output unit. The input unit is configured to receive image data acquired by a spectral CT scanner. The processing unit is configured to perform an analysis of a contrast enhancement in a region of interest in the acquired image data, and to compare the contrast enhancement of the region of interest to a desired contrast enhancement as defined by an exam protocol to determine whether the contrast enhancement of the region of interest is sufficient. In response to determining that there is an insufficient contrast enhancement of the region of interest, the processing unit is configured to generate a trigger signal, which is usable to trigger the spectral CT scanner to export spectral data. The output unit is configured to output the generated trigger signal.

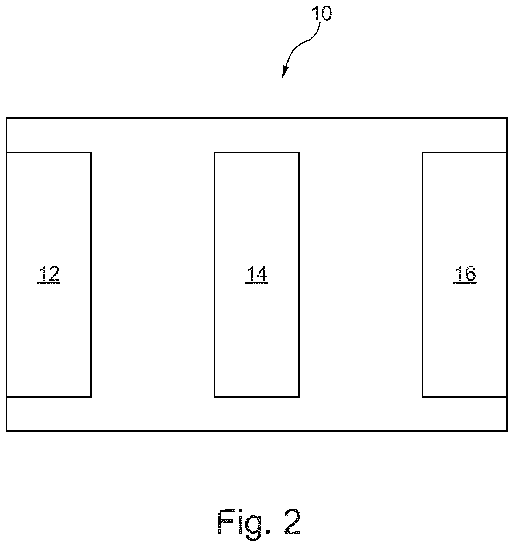

In other words, an image quality assurance device is provided that performs an analysis of the contrast enhancement in the conventional scan given the imaging data as well as the clinical indication. Upon detection of sub-optimal contrast enhancement, the image quality assurance device triggers the additional export of the spectral data to a data repository, such as a picture archiving and communication system (PACS). A notification, e.g., alert, may be created for a user, e.g., a radiologist, to read the additional images for better diagnosis. The proposed image quality assurance device may allow performing conventional CT acquisitions on a spectral system and only retrospectively saving of the spectral data, thus enabling a more confident diagnosis of the clinical questions. This will be described in detail hereinafter and in particular with respect to the embodiment shown in FIG. 2.

According to an exemplary embodiment of the first aspect of the present invention, in response to determining that there is an insufficient contrast enhancement of the region of interest, the processing unit is further configured to generate a notification indicating that the contrast enhancement of the target anatomy is insufficient.

For example, the notification may provide visual and/or audible indications in the form of e.g., LEDs, messages on a display, and/or sounds.

According to an exemplary embodiment of the first aspect of the present invention, the notification further comprises one or more of the following information: a recommended reconstruction energy level yielding a sufficient contrast enhancement, and a quantification of the determined contrast enhancement of the region of interest.

Accordingly, such notification may contain additional information such as a recommended reconstruction energy level yielding an improved enhancement pattern, and/or quantifications of measured enhancement.

According to an exemplary embodiment of the first aspect of the present invention, the processing unit is further configured to reconstruct an improved diagnostic image using the acquired image data and the spectral data.

Accordingly, the information of the conventional and spectral data may be used to reconstruct an improved diagnostic image, which may be stored in a data repository along the conventional images for reading.

According to an exemplary embodiment of the first aspect of the present invention, the processing unit is configured to perform an image segmentation operation on the acquired image data to determine the region of interest.

According to an exemplary embodiment of the first aspect of the present invention, the contrast enhancement includes an enhancement pattern of the region of interest, wherein the enhancement pattern is a distribution of the contrast enhancement in two or more anatomic structures, spatial regions, and/or over time.

According to an exemplary embodiment of the first aspect of the present invention, the region of interest comprises a region expected to be enhanced and/or a region expected to remain unenhanced.

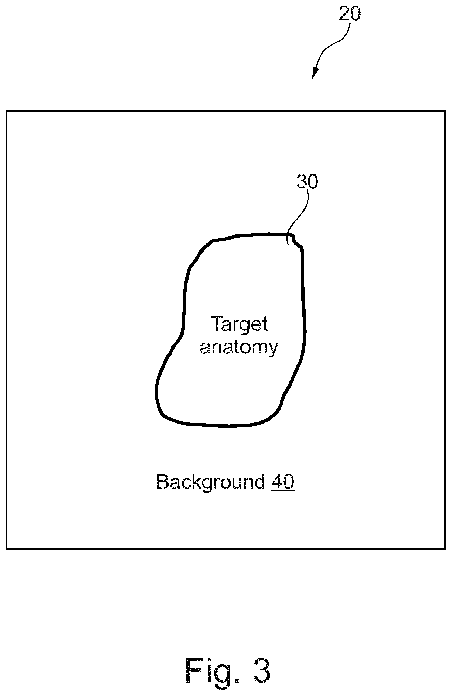

FIG. 3 schematically illustrates an exemplary imaging volume with a region expected to be enhanced, e.g., target anatomy, and a region expected to remain unenhanced, e.g., background. According to an exemplary embodiment of the first aspect of the present invention, the spectral data is in a form of Spectral Base Images (SBI).

According to a second aspect of present invention, there is provided a CT imaging system, which comprises a spectral CT scanner configured to acquire image data of a subject, and the imaging quality assurance device according the first aspect and any associated example.

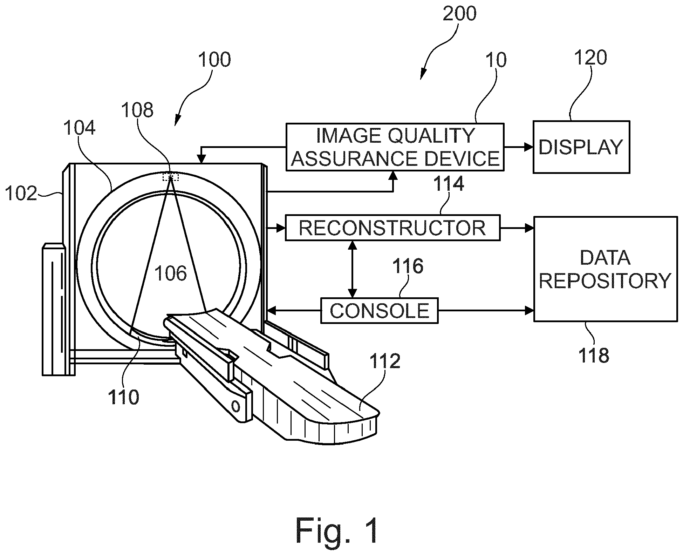

This will be described in detail hereinafter and in particular with respect to the embodiment shown in FIG. 1.

According to a third aspect of the present invention, there is provided an image quality assurance method, which comprises:
a) receiving, by a processor, image data acquired by a spectral Computed Tomography, CT, scanner;
b) performing, by the processor, an analysis of a contrast enhancement in a region of interest in the acquired image data;
c) comparing, by the processor, the contrast enhancement of the region of interest to a desired contrast enhancement as defined by an exam protocol to determine whether the contrast enhancement of the region of interest is sufficient; and
d) in response to determining that there is an insufficient contrast enhancement of the region of interest, generating, by the processor, a trigger signal, which is usable to trigger the spectral CT scanner to export spectral data.

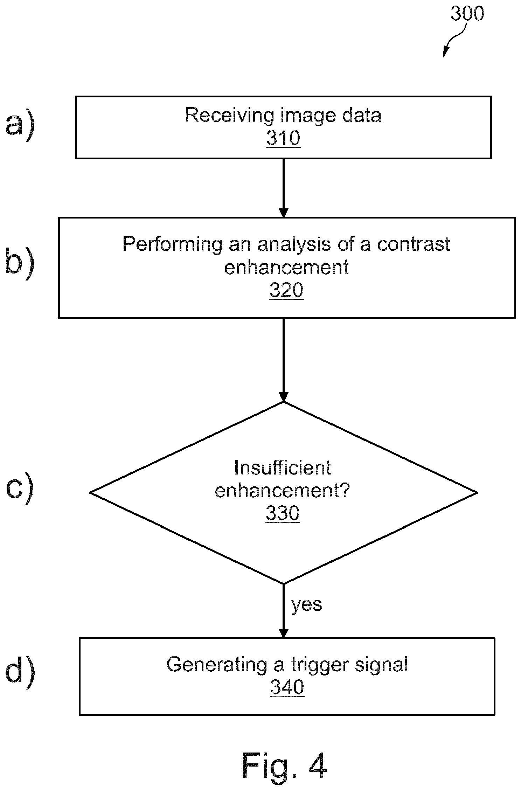

This will be described in detail hereinafter and in particular with respect to the embodiment shown in FIG. 4.

According to an exemplary embodiment of the third aspect of the present invention, the image quality assurance method further comprises the step of in response to determining that there is an insufficient contrast enhancement of the region of interest, generating a notification indicating that the contrast enhancement of the target anatomy is insufficient.

According to an exemplary embodiment of the third aspect of the present invention, the notification comprises one or more of the following information: a recommended reconstruction energy level yielding a sufficient contrast enhancement, and a quantification of the determined contrast enhancement of the region of interest.

According to an exemplary embodiment of the third aspect of the present invention, the image quality assurance method further comprises the step of reconstructing an improved diagnostic image using the acquired image data and the spectra data.

According to another aspect of the present invention, there is provide a computer program product comprising instructions which, when the program is executed by a processor, cause the processor to carry out the steps of the method of the first aspect of the associated example.

According to a further aspect of the present invention, there is provided a computer-readable medium having stored thereon the computer program product.

As used herein, the term "unit" may refer to, be part of, or include an Application Specific Integrated Circuit (ASIC), an electronic circuit, a processor (shared, dedicated, or group) and/or memory (shared, dedicated, or group) that execute one or more software or firmware programs, a combinational logical circuit, and/or other suitable components that provide the described functionality

### BRIEF DESCRIPTION OF THE DRAWINGS

These and other aspects of the invention will be apparent from and elucidated further with reference to the embodiments described by way of examples in the following description and with reference to the accompanying drawings, in which
FIG. 1 schematically illustrates an exemplary imaging system.
FIG. 2 schematically illustrates an exemplary image quality assurance device.
FIG. 3 schematically illustrates an exemplary imaging volume.
FIG. 4 illustrates an exemplary image quality assurance method.
It should be noted that the figures are purely diagrammatic and not drawn to scale. In the figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

FIG. 1 illustrates an exemplary imaging system 200 that includes a spectral CT scanner 100 configured to acquire image data of a subject (e.g., human or animal). In the example illustrated in FIG. 1, the spectral CT scanner 100 comprises a stationary gantry 102 and a rotating gantry 104, which is rotatably supported by the stationary gantry 102. The rotating gantry 104 rotates around an examination region 106 about a longitudinal or z-axis.

The spectral CT scanner 100 includes at least one radiation source 108, such as an x-ray tube, that is supported by the rotating gantry 104 and which rotates with the rotating gantry 104 about the examination region 106. The at least one radiation source 108 emits radiation that traverses the examination region 106. If there are two or more radiation sources 108, each source may be configured to emit radiation having a different mean emission spectrum. Additionally or alternatively, one or more of the at least two sources 108 may be configured to controllably switch between at least two different emission voltages (kVp's) during scanning multiple sources and/or kVp switching can be used for spectral CT acquisitions.

The spectral CT scanner 100 further includes a radiation sensitive detector array 110, which is located opposite the at least one radiation source 108, across the examination region 106. The radiation sensitive detector array 110 may include an array of detector pixels that detect radiation traversing the examination region 106 and generate projection data indicative thereof. In some examples, the radiation sensitive detector array 110 may include energy-resolving detectors such as direct conversion detectors and/or a scintillator-based multi-spectral detector that includes at least two scintillators with different x-ray energy sensitivities respectively optically affixed to at least two photosensors with corresponding optical sensitivities (e.g., double decker or layer detector). The energy-resolving detectors may be used for spectral CT acquisitions. In some examples, the radiation sensitive detector array 110 may include photon-counting detector pixels (e.g., CdTe, CdZnTe, etc.). The photon-counting detector pixels are configured to acquire the spectral nature of the x-ray source rather than the energy integration nature in acquiring data. To obtain the spectral nature of the transmitted X-ray data, the photo counting detector pixels split the x-ray beam into its component energies or spectrum bins and counts a number of photons in each of the bins.

The spectral CT scanner 100 may further comprise a subject support 112, such as a couch, to support a subject (e.g., a human or animal) or object in the examination region 106 and can be used to position the subject with respect to x, y, and/or z axes and the examination 106 before, during and/or after scanning.

The imaging system 200 may further comprise a reconstructor 114 to reconstruct the projection data and to generate volumetric image data indicative of the examination region 106 and the portion of the object or subject therein. Where spectral data is acquired, the reconstructor 112 may reconstruct individual spectral images for each of the different energy ranges and/or combination images based on the individual spectral images corresponding to two or more of the different energy ranges. The reconstructor 114 may also employ conventional non-spectral reconstruction algorithms.

The imaging system 200 may further comprise an operator console 116, which may include an output device such as a display and an input device such as a keyboard, mouse, and/or the like. Software resident on the console 116 may allow the operator to control the operation of the spectral CT scanner 100, for example, allowing the operator to select a spectral imaging protocol, initiate scanning, etc.

A data repository 118 can be used to store the reconstructed images and may be accessed by one or more of the console 116 and/or other device. The data repository 118 can be local to the imaging system 200, remote from the imaging system 200, distributed, etc. The data repository 118 may include a database, a server, PACS, radiology information system (RIS), a hospital information system (HIS), an electronic medical record (EMR), and/or other electronic storage device or memory.

As described above, contrast-enhanced CT exams allow to use lower quantities of contrast agent when performing spectral acquisitions. Unfortunately, even though the scanner has spectral capabilities, many of the scans performed on those machines are not set up to record the spectral information along with the imaging data, basically throwing away the ability to reconstruct mono-energetic images.

In order to solve the above problem, an image quality assurance device 10 is provided. As shown in FIG. 1, the image quality assurance device 10 is configured to receive image data acquired by the spectral CT scanner 100 via a wired or wireless connection, including USB, Ethernet, Bluetooth, Wi-Fi, and others. The image quality assurance device 10 is configured to perform an analysis of a contrast enhancement in a region of interest in the acquired image data, and to compare the contrast enhancement of the region of interest to a desired contrast enhancement as defined by an exam protocol to determine whether the contrast enhancement of the region of interest is sufficient. In response to determining that there is an insufficient contrast enhancement of the region of interest, the image quality assurance device 10 is configured to generate a trigger signal and output the generated trigger signal to the spectral CT scanner 100 via a wired or wireless connection, including USB, Ethernet, Bluetooth, Wi-Fi, and others. The trigger signal is configured to trigger the spectral CT scanner to export spectral data e.g., in the form of Spectral Base Images (SBI). The image quality assurance device 10 thus allows performing conventional CT acquisitions on spectral system and only retrospectively storing or saving the spectral data e.g., in the form of SBI, thus enabling a more confident diagnosis of the clinical question. In the example of contrast, the image quality assurance device 10 may analyze the "conventionally" acquired imaging data for contrast enhancement in the desired anatomy, e.g., tissue/vessels. If a low enhancement is detected by the module, the spectral data is reconstructed and stored in the data repository 118, such as PACS, along with the conventional imaging results. Consequently, when reading the case, a user, e.g., radiologist, has the option to use the spectral information to reconstruct additional, mono-energetic images with a brighter enhancement characteristics. While FIG. 1 may show the image quality assurance device 10 as a stand-alone device, it will be appreciated that in another implementation, the image quality assurance device 10 may be implemented as or in other devices, such as via the console 116, e.g., as a software resident on the console 116.

FIG. 2 illustrates an example of the image quality assurance device 10. As shown in FIG. 2, the image quality assurance device 10 comprises an input unit 12, a processing unit 14, and an output unit 16.

In general, the image quality assurance device 10 may comprise various physical and/or logical components for communicating and manipulating information, which may be implemented as hardware components (e.g., computing devices, processors, logic devices), executable computer program instructions (e.g., firmware, software) to be executed by various hardware components, or any combination thereof, as desired for a given set of design parameters or performance constraints. Although FIG. 2 may show a limited number of components by way of example, it can be appreciated that a greater or a fewer number of components may be employed for a given implementation.

In some implementations, the image quality assurance device 10 may be embodied as, or in, a device or apparatus, such as a server, workstation, or mobile device. The apparatus 10 may comprise one or more microprocessors or computer processors, which execute appropriate software. The processing unit 14 of the image quality assurance device 10 may be embodied by one or more of these processors. The software may have been downloaded and/or stored in a corresponding memory, e.g., a volatile memory such as RAM or a non-volatile memory such as flash. The software may comprise instructions configuring the one or more processors to perform the functions as described herein.

It is noted that the image quality assurance device 10 may be implemented with or without employing a processor, and also may be implemented as a combination of dedicated hardware to perform some functions and a processor (e.g., one or more programmed microprocessors and associated circuitry) to perform other functions. For example, the functional units of the image quality assurance device 10, e.g., the input unit 12, the one or more processing units 14, and the output unit 16 may be implemented in the device or apparatus in the form of programmable logic, e.g., as a Field-Programmable Gate Array (FPGA). In general, each functional unit of the apparatus may be implemented in the form of a circuit.

In some implementations, the image quality assurance device 10 may also be implemented in a distributed manner. For example, some or all units of the apparatus 10 may be arranged as separate modules in a distributed architecture and connected in a suitable communication network, such as a 3rd Generation Partnership Project (3GPP) network, a Long Term Evolution (LTE) network, Internet, LAN (Local Area Network), Wireless LAN (Local Area Network), WAN (Wide Area Network), and the like.

The input unit 12 is configured to receive the image data acquired by the spectral CT scanner 100. The input unit 12 may be implemented as a wired interface, such as an Ethernet interface, or a USB interface, or a wireless interface, such as Wi-Fi, Bluetooth, or any comparable interface (e.g., 3GPP network interface, LTE network interface, etc.) enabling data transfer between the spectral CT scanner 100 and the image quality assurance device 10.

The processing unit 14 is configured to perform an analysis of a contrast enhancement in a region of interest in the acquired image data, and to compare the contrast enhancement of the region of interest to a desired contrast enhancement as defined by an exam protocol to determine whether the contrast enhancement of the region of interest is sufficient. The intensity of the contrast enhancement may be measured in Hounsfield units (HU), which may be compared with a desired contrast enhancement as defined by an exam protocol. It is noted that the region of interest may comprise a region expected to be enhanced, such as a target anatomy shown in FIG. 3, which may be vessels, organs, pathological tissues (e.g., tumors), etc. The region of interest may alternatively or additionally comprise a region expected to remain unenhanced, such as the background region shown in FIG. 3.

In some implementations, the processing unit 14 may be configured to perform an image segmentation operation on the acquired image data to determine the region of interest. In some examples, the processing unit 14 may apply an atlas-based method for automatic segmentation. The atlas-based methods propagate the predefined structure contours to the images to be segmented using image registration. In some examples, the processing unit 14 may apply a model-based method which makes use of statistical shape models for automated segmentation. In some examples, the processing unit 14 may apply a deep-learning-based medical image segmentation method. Examples of the deep-learning-based medical image segmentation methods may include, but are not limited to, convolutional neural network (CNN), U-Net, Mask R-CNN, RefineNet, and DeconvNet. FIG. 3 illustrates an example of a target anatomy 30, such as blood vessels, organs, segmented from an imaging volume in the acquired image data 20. The analysis of the contrast enhancement may be performed in a region expected to be enhanced, such as the target anatomy 30, and/or in a region expected to remain unenhanced, such as the background 40.

There are different types of contrast enhancements depending of the desired enhancement. For example in angiographies, the target anatomy is the blood in the vessels. The scan is ideally performed quite quickly after injection to allow the contrast agent to reach the target region but to not "leak" into the surrounding tissues and also enhancing the "background". Because timing is critical in these applications, missing the ideal acquisition window can lead to insufficient contrast agent in the region of interest. For other applications, the desired effect is for the contrast agent to saturate the entire target anatomy. In those cases, there is a usually a longer scan delay and the effect of missed timing is more in the inhomogenous enhancement realm. Therefore, the contrast enhancement may include an enhancement pattern of the region of interest. The enhancement pattern may comprise a distribution of the contrast enhancement in two or more anatomic structures and/or spatial regions. Alternatively or additionally, the enhancement pattern may comprise a distribution of the contrast enhancement over time, such as dynamic enhancement patterns of intrahepatic cholangiocarcinoma in cirrhosis. Therefore, in some implementations, the processing unit 14 may be configured to measure the actual enhancement pattern, which may be dependent on the timing and the organ, in the segmented region of interest and to compare it to the desired enhancement pattern as defined by the exam protocol.

Turning back to FIG. 2, in response to determining that there is an insufficient contrast enhancement of the region of interest, the processing unit 14 is configured to generate a trigger signal, and to provide the trigger signal via the output unit 16 to the spectral CT scanner 100. The output unit 16 may be implemented as a wired interface, such as an Ethernet interface, or a USB interface, or a wireless interface, such as Wi-Fi, Bluetooth, or any comparable interface (e.g., 3GPP network interface, LTE network interface, etc.) enabling data transfer between the spectral CT scanner 100 and the image quality assurance device 10. The trigger signal is used to trigger the spectral CT scanner to export spectral data, e.g., SBI spectral information. Therefore, as shown in FIG. 1, in case where an insufficient contrast enhancement, such as an insufficient contrast enhancement pattern, is detected, a trigger signal is generated by the image quality assurance device 10, which triggers an additional export of the spectral data, e.g., SBI spectral information, from the spectral CT scanner 100 to the data repository 118, such as PACS.

In addition to the spectral data export, the processing unit 14 of the image quality assurance device 10 may be further configured to generate a notification for a radiologist to read additional available images for better diagnosis. The notification may provide visual and/or audible indications in the form of e.g., LEDs, messages on a display, and/or sounds. For example, the notification may be shown on a display 120, which may be a display of the operator console 116. In this way, the radiologist may read the case more confidently and mitigate the effects of the insufficient contrast enhancement. Such notifications may contain additional information such as a recommended reconstruction energy level yielding a sufficient contrast enhancement, and/or a quantification of the determined contrast enhancement of the region of interest.

In some implementations, the information of the conventional and spectral data may be used to reconstruct an improved diagnostic image, which may be stored in the data repository 118, such as PACS, along the conventional image for reading. The image reconstruction may be carried out by the reconstructor 114 shown in FIG. 1 or the processing unit 14 of the image quality assurance device 10.

FIG. 4 illustrates a flowchart describing an exemplary image quality assurance method 300.

At block 310, i.e., step a), the method 300 comprises the step of receiving image data acquired by a spectral CT scanner. For example, as shown in FIG. 1, the image quality assurance device 10 may receive image data acquired by the spectral CT scanner 100 through a wired or wireless connection, including USB, Ethernet, Bluetooth, Wi-Fi, and others.

At block 320, i.e., step b), the method 300 comprises the step of performing an analysis of a contrast enhancement in a region of interest in the acquired image data. The intensity of the contrast enhancement may be measured in HU values.

In some examples, the region of interest may comprise a region expected to be enhanced, such as a target anatomy like blood vessels, and organs. In some examples, the region of interest may comprise a region expected to remain unenhanced, such as background in the imaging volume. The region expected to remain unenhanced may alternatively or additionally include other anatomical structures. For example, in angiographies, the vessels are expected to be enhanced, but the agent is not supposed to be leaked into the surrounding soft tissue yet. The surrounding soft tissue may be considered as the region expected to remain unenhanced in this example. In some examples, the region of interest may comprise both the region expected to be enhanced and the region expected to remain unenhanced. The desired region of interest may be segmented from the imaging volume using various technologies, such as atlas-based method for automatic segmentation, model-based segmentation method, and deep-learning-based medical image segmentation method.

In some examples, an actual enhancement pattern may be measured in the segmented region of interest. The enhancement pattern may comprise a distribution of the contrast enhancement in two or more anatomic structures, spatial regions, and/or over time. As an example, the enhancement pattern may comprise a distribution of the contrast enhancement determined by comparing two images acquired from a pre-contrast scan and a post-contrast scan.

At block 330, i.e., step c), the method 300 further comprises the step of comparing the contrast enhancement of the region of interest to a desired contrast enhancement as defined by an exam protocol to determine whether the contrast enhancement of the region of interest is sufficient.

For example, the intensity of the contrast enhancement may be measured in HU values, which may be compared to the desired contrast enhancement as defined by an exam protocol to determine whether the contrast enhancement of the region of interest is sufficient.

If it is determined that there is an insufficient contrast enhancement, such as an insufficient enhancement pattern, step d) is performed at block 340. At block 340, a trigger signal is generated, which is usable to trigger the spectral CT scanner to export spectral data, such as SBI spectral information.

In addition to the step of generating the trigger signal, the method 300 may further comprise the step of generating a notification indicating that the contrast enhancement of the target anatomy is insufficient. The notification may additionally comprise a recommended reconstruction energy level yielding a sufficient contrast enhancement, and/or a quantification of the determined contrast enhancement of the region of interest.

In some examples, the method 300 may further comprise the step of reconstructing an improved diagnostic image using the acquired image data and the spectra data.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An image quality assurance device (10), comprising:
- an input unit (12);
- a processing unit (14); and
- an output unit (16);
wherein the input unit is configured to receive image data acquired by a spectral Computed Tomography, CT, scanner;
wherein the processing unit is configured to perform an analysis of a contrast enhancement in a region of interest in the acquired image data, and to compare the contrast enhancement of the region of interest to a desired contrast enhancement as defined by an exam protocol to determine whether the contrast enhancement of the region of interest is sufficient;
wherein in response to determining that there is an insufficient contrast enhancement of the region of interest, the processing unit is configured to generate a trigger signal, which is usable to trigger the spectral CT scanner to export spectral data; and
wherein the output unit is configured to output the generated trigger signal.

2. The image quality assurance device according to claim 1,
wherein in response to determining that there is an insufficient contrast enhancement of the region of interest, the processing unit is further configured to generate a notification indicating that the contrast enhancement of the target anatomy is insufficient.

3. The image quality assurance device according to claim 2,
wherein the notification further comprises one or more of the following information:
- a recommended reconstruction energy level yielding a sufficient contrast enhancement; and
- a quantification of the determined contrast enhancement of the region of interest.

4. The image quality assurance device according to any one of the preceding claims,
wherein the processing unit is further configured to reconstruct an improved diagnostic image using the acquired image data and the spectral data.

5. The image quality assurance device according to any one of the preceding claims,
wherein the processing unit is configured to perform an image segmentation operation on the acquired image data to determine the region of interest.

6. The image quality assurance device according to any one of the preceding claims,
wherein the contrast enhancement includes an enhancement pattern of the region of interest, wherein the enhancement pattern comprises a distribution of the contrast enhancement in two or more anatomic structures, spatial regions, and/or over time.

7. The image quality assurance device according to any one of the preceding claims,
wherein the region of interest comprises a region expected to be enhanced and/or a region expected to remain unenhanced.

8. The image quality assurance device according to any one of the preceding claims,
wherein the spectral data is in a form of Spectral Base Images, SBI.

9. An imaging system (100), comprising:
- a spectral Computed Tomography, CT, scanner (100) configured to acquire image data of a subject; and
- the imaging quality assurance device (10) according to any one of the preceding claims.

10. An image quality assurance method (300), comprising:
a) receiving (310), by a processor, image data acquired by a spectral Computed Tomography, CT, scanner;
b) performing (320), by the processor, an analysis of a contrast enhancement in a region of interest in the acquired image data;
c) comparing (330), by the processor, the contrast enhancement of the region of interest to a desired contrast enhancement as defined by an exam protocol to determine whether the contrast enhancement of the region of interest is sufficient; and
d) in response to determining that there is an insufficient contrast enhancement of the region of interest, generating (340), by the processor, a trigger signal, which is usable to trigger the spectral CT scanner to export spectral data, SBI.

11. The image quality assurance method according to claim 10, further comprising:
- in response to determining that there is an insufficient contrast enhancement of the region of interest, generating a notification indicating that the contrast enhancement of the target anatomy is insufficient.

12. The image quality assurance method according to claim 11,
wherein the notification comprises one or more of the following information:
- a recommended reconstruction energy level yielding a sufficient contrast enhancement; and
- a quantification of the determined contrast enhancement of the region of interest.

13. The image quality assurance method according to any one of claims 10 to 12, further comprising:
- reconstructing an improved diagnostic image using the acquired image data and the spectra data.

14. A computer program product comprising instructions which, when the program is executed by a processor, cause the processor to carry out the steps of the method of any one of claims 10 to 13.

15. A computer-readable medium having stored thereon the computer program product of claim 14.
